# EUROPEAN PATENT SPECIFICATION

(11) **EP 2 717 817 B1**
(45) Date of publication and mention of the grant of the patent: **12.07.2017**
(21) Application number: 11751593.2
(22) Date of filing: 30.08.2011
(51) Int. Cl.: A61F 13/493, A61F 13/80, A61F 13/505, A61F 13/49, A61F 13/494, A61F 13/15, A61F 13/56, A61F 13/84

(54) **DIAPER SHEATH**
WINDELHÜLLE
GAINE DE COUCHE

(30) Priority: 09.06.2011 US 201113157190
(43) Date of publication of application: 16.04.2014
(73) Proprietor: Pure Precision Limited, Tortola (VG)
(72) Inventor: TOURNIER, Gaelle, Road Town, Tortola (VG)
(74) Representative: Bergenstråhle Group AB
(86) International application number: PCT/EP2011/064882
(87) International publication number: WO 2012/167844

(56) References cited:
- WO-A1-98/29080
- CN-Y- 2 313 397
- GB-A- 2 468 724
- US-A1- 2002 010 452
- US-A1- 2009 187 156
- US-A1- 2009 240 228

## Description

### FIELD

The present invention relates generally to diapers, and more particularly to a diaper having continuously adjustable leg openings.

### BACKGROUND

Presently, diapers for infants (or others such as children, adults or animals) include cloth diapers, disposable diapers and all-in-one diapers. The manufacture, use and disposal of any diaper presents health and sanitation issues, energy and environmental cost issues and economic issues.

Conventional diapers with leg opening adjusting members may have an area contacting the skin of the wearer that is too large. Also, the material of some conventional adjustment members may be hard and uncomfortable for the wearer.

US 2009/240228 A1 discloses an absorbent article including a chassis having a front section, rear section and a crotch section extending between the front section and the rear section and a traditional tensioner to hold the article in place. Such tensioners are difficult to fix in the correct position causing discomfort and potential leakage.

### SUMMARY

In addition to the above issues, a technical problem with conventional diapers can be balancing the comfort of the diaper wearer with a need to prevent leakage from the diaper. A diaper needs to be snug enough to ensure retention of solid and liquid waste and to prevent leakage, but not so snug as to be uncomfortable or hamper blood circulation in the wearer's legs or torso, or to create high humidity or moisture within the diaper. Discomfort or hampering of blood circulation can be caused by conventional, nonadjustable elastic leg openings. The hook and loop fasteners provided on conventional diapers may only allow for waist band adjustment may not provide for adjustment of the leg openings.

Embodiments of the present invention can solve the above-mentioned problem by providing, for example, a diaper sheath that has continuously adjustable leg openings that can be used to balance wearer comfort with snugness to prevent leakage. For example, one or more embodiments can provide, among other things, the following features that may provide a solution to the technical problem and other issues discussed above:
(1) leg openings having a size that can be adjusted by moving a respective adjustment member to a desired location on each intermediate strip in the diaper sheath of the present invention, so the diaper can be more comfortable for the wearer;
(2) an intermediate pocket flap, front flap or back flap that can cover the intermediate strips inside the diaper sheath so that the intermediate strips and adjustment members do not contact the skin of the wearer directly and therefore can help reduce or eliminate any uncomfortable feeling; and
(3) a way to accurately and quickly determine the proper position of the adjustment members using measuring indicators on the intermediate strip.

The present invention was conceived in light of the above technical problems and limitations, among other things.

In one embodiment, a diaper sheath includes a shell having an inner panel and an outer panel, with each of the inner and outer panels having a front portion and a back portion, and with each of the panels being attached peripherally one to the other in matched contour to form a front edge, a back edge, and a first side edge and a second side edge. The diaper sheath can also include a first intermediate pocket and a second intermediate pocket set along the first side edge and the second side edge respectively, each intermediate pocket having at least one opening. The diaper sheath can also include a first intermediate strip and a second intermediate strip set inside each of the first and second intermediate pockets respectively, the first and second intermediate strips each having an anchor portion attached to an attachment ring, and an elongated portion having a loop formed through a slide, the loop passing through the attachment ring and having a continuously adjustable length selected by moving the slide to a desired position along the elongated portion.

In various embodiments, the openings of the diaper sheath are set in a substantially middle portion of the intermediate pocket. Also, a portion of the intermediate strip can be exposed outside the intermediate pockets through the intermediate pocket openings. The diaper sheath can also include intermediate pocket flaps positioned at the opening(s) of the intermediate pocket, the intermediate pocket openings are covered by the intermediate pocket flaps. The diaper sheath can have two intermediate pocket openings on each intermediate pocket.

In various embodiments of the diaper sheath, the front portion of the inner panel can have a front opening disposed thereon. The diaper sheath can also include a front flap attached to the front portion of the inner panel, and the front opening can be covered by the front flap. The intermediate strips and the intermediate pocket openings can be covered by the front flap. Also, the back portion of the inner panel can have a back opening.

In other embodiments, the diaper sheath can include a back flap attached to the back portion of the inner panel, which covers the back opening. The diaper sheath can have intermediate strips and the intermediate pocket openings that are covered by the back flap. The slide can be a spring-loaded stopper and each intermediate strip can be inserted through a respective stopper so that the position of the intermediate strip can be continuously adjusted. The stoppers can be placed within the intermediate pocket or disposed at the opening of the intermediate pocket.

In still other embodiments, the inner panel can utilize a laminated liquid permeable material and the outer panel utilizes a laminated liquid impervious material. The laminated liquid impervious material can be a layer of laminated organic cotton on the outer surface of the outer panel. The inner panel can be constructed of fleece material made of 100% polyester.

Yet other embodiments can include a back elastic strip attached to the inner and outer panels along the edge of the back portion. An adjustment member can be disposed so as to slidably engage the back elastic strip. The first and second intermediate strips can be selected from the group consisting of elastic band, string, rope or thread.

In some embodiments, the first and second intermediate strips are each a solid elastic band. One or more embodiments can include two measuring indicators each disposed on a respective intermediate strip. The measuring indicator can be selected from the group consisting of scale marks, numbers or letters.

The diaper sheath can include a pad fully or partially inserted between the inner and outer panels. The pad can be a removable, reusable, absorbent pad. Also, the pad can be disposable.

The intermediate strip can be elastic band, string, rope, thread or other suitable material. The diaper (or diaper sheath) can include two measuring indicators set on each of the intermediate strips respectively. The measuring indicator can be scale marks, numbers, letters or any other suitable measuring indicators. In an embodiment with holes in the intermediate strips, the measuring indicator can be disposed beside the hole, or holes. In various embodiments, the intermediate strips are solid and do not have holes.

Still another embodiment includes a diaper having a diaper sheath according to at least one embodiment and a pad that can be fully or partially inserted between the inner and outer panels. The pad can be a removable, reusable, and/or absorbent pad. The pad can also be a disposable pad.

### BRIEF DESCRIPTION OF THE DRAWINGS

For a further understanding of the present invention, reference should be made to the following detailed description taken in conjunction with the accompanying drawings, wherein:
Fig. 1 is an inside view of an exemplary diaper sheath in accordance with at least one embodiment;
Fig. 2 is an outside view of an exemplary diaper sheath in accordance with at least one embodiment;
Fig. 3 is an inside view of an exemplary diaper sheath showing a pad in accordance with at least one embodiment;
Fig. 4 is a cross-sectional view of an exemplary intermediate strip in accordance with at least one embodiment;
Fig. 5 is a cross-sectional view of an exemplary intermediate strip in accordance with at least one embodiment;
Fig. 6 is a view of an exemplary intermediate pocket flap in accordance with at least one embodiment;
Fig. 7 is a view of an exemplary intermediate pocket flap in accordance with at least one embodiment;
Fig. 8 is a view of an exemplary measuring indicator in accordance with at least one embodiment;
Fig. 9 is a view of an exemplary measuring indicator in accordance with at least one embodiment;
Fig. 10 is a view of an exemplary intermediate strip with the measuring indicator in accordance with at least one embodiment;
Fig. 11 is a view of an exemplary intermediate pocket with a stopper in accordance with at least one embodiment;
Fig. 12 is a view of an exemplary intermediate strip with a stopper in accordance with at least one embodiment;
Fig. 13 is a view of an exemplary diaper sheath in accordance with at least one embodiment;
Fig. 14 is a cross-sectional view of an exemplary intermediate strip with a stopper in accordance with at least one embodiment;
Fig. 15 is a cross-sectional view of an exemplary intermediate pocket comprising front and back snaps in accordance with at least one embodiment;
Fig. 16 is an outside view of an exemplary diaper sheath with an outside front flap and opening in accordance with at least one embodiment;
Fig. 17 is an outside view of an exemplary diaper sheath with an outside front flap open showing the interior of a front opening in accordance with at least one embodiment;
Figs. 18 and 19 are views of an exemplary diaper sheath showing continuously adjustable leg opening adjustment members disposed for access from within a front opening in accordance with at least one embodiment; and
Figs. 20 and 21 are views of an exemplary diaper sheath showing continuously adjustable leg opening adjustment members disposed for access from exterior front openings in accordance with at least one embodiment.

### DETAILED DESCRIPTION

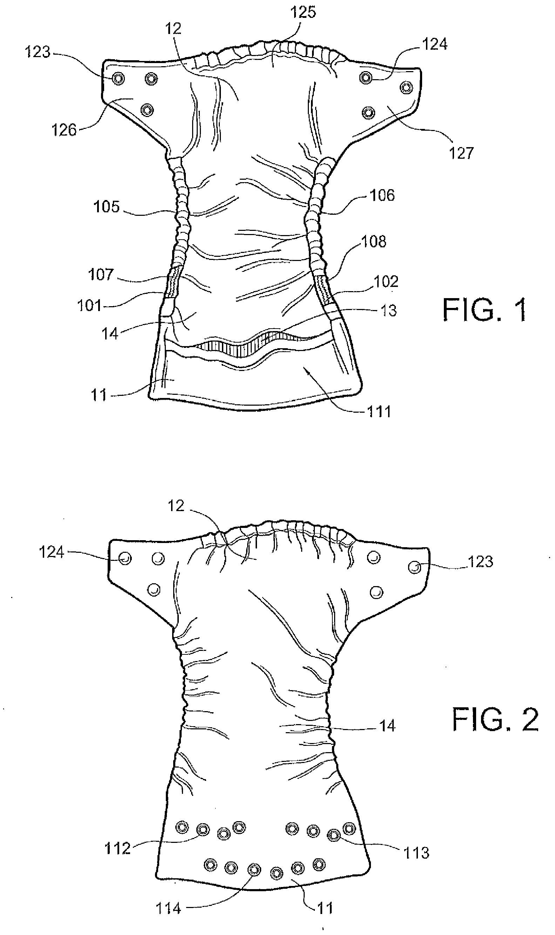

An exemplary diaper is shown in Figs. 1 and 2 as viewed from the inside and outside, respectively. In particular, the diaper includes an inner panel and an outer panel, each inner and outer panel having a front portion 11 and a back portion 12 connected by an intermediate portion 14. The front portion 11 of the inner panel has a front opening 13. The inner and outer panels are attached together peripherally in matched contour. The back portion 12 has two elongated wings 126, 127. The front portion 11 has a plurality of front snaps 112, 113, 114 attached to the front portion 11 of the outer panel. The back portion 12 has a plurality of back snaps 123, 124 attached to the two elongated wings 126, 127, respectively. Each of the plurality of back snaps 123, 124 is cooperatively fastenable with one of the front snaps 112, 113, 114.

The diaper also includes a front flap 111 attached to the front portion 11 of the inner panel. The front opening 13 is covered by the front flap 111. The diaper also includes two intermediate pockets 105, 106, which are set (or disposed) along respective side edges of intermediate portion 14. There is one intermediate pocket opening 107, 108 on each respective intermediate pocket 105, 106. As shown in this embodiment, the intermediate pocket openings 107, 108 are set in the middle portion of the intermediate pockets 105, 106. The diaper also includes intermediate elastic strips 101, 102, one end of which are loosely hidden inside the intermediate pockets 105, 106 and can go through the intermediate strip openings 107, 108. The portion of the intermediate strips 101, 102 that are positioned at the intermediate pocket openings 107, 108 can be exposed outside the intermediate pockets 105, 106. The intermediate strips 101, 102 can be elastic bands or other elastic or stretchable material. Alternatively, the intermediate strips 101, 102 can be ropes, threads, strings or other material not having elasticity.

Also, the intermediate pocket openings 107, 108 can be formed narrow, so that intermediate strips 101,102 will not be exposed outside the intermediate pockets 105, 106.

The diaper can include intermediate buttons 103, 104 disposed on the inner surface of the intermediate pockets 105, 106.

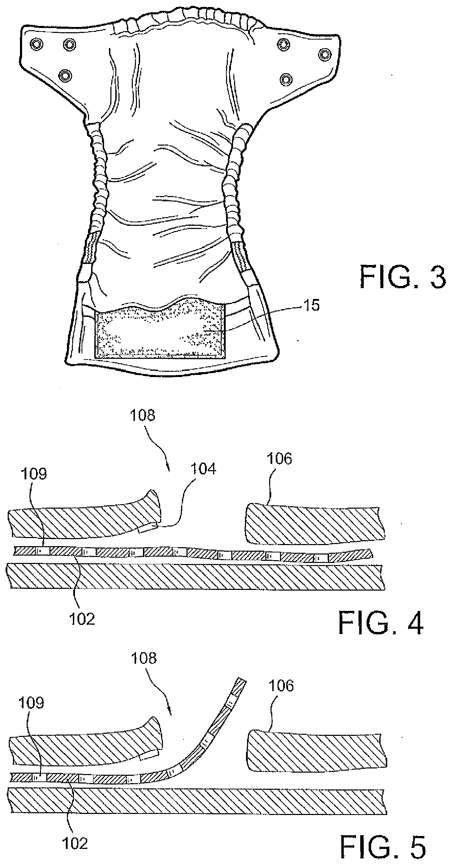

Taking the intermediate pocket 106 on one side as an example, as shown in Figs. 4 and 5, the intermediate strip 102 can have a plurality of intermediate holes 109 set in a spaced apart arrangement. The intermediate button 104 is cooperatively fastenable with one of the intermediate holes 109. During usage, a user can draw the intermediate strip 102 out via the intermediate pocket opening 108, fasten the suitable intermediate hole 109 onto the intermediate button 104, and put the loose end of the intermediate strip 102 back into the intermediate pocket 106 via the intermediate pocket opening 108. By adjusting the position on the intermediate strips 101, 102 where the intermediate button is fastened, the extension of the elastic leg line can be easily adjusted, and the diaper sheath can be more fitting with the skin of the baby and more comfortable for the baby.

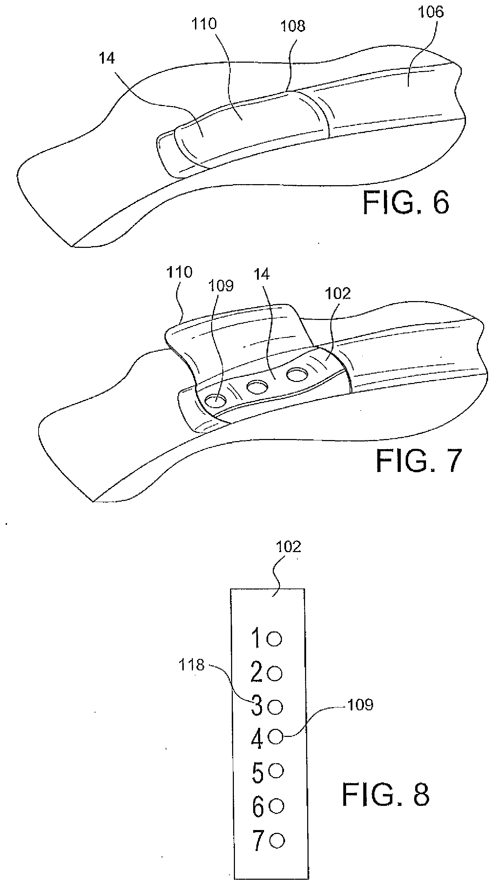

In another embodiment of this invention, there is an intermediate pocket flap 110 on each intermediate pocket opening 107, 108 that covers the portion of intermediate strips 101, 102 (refer to Figs. 6 and 7) exposed outside the intermediate strips 101, 102. This structure can also prevent the intermediate strips 101, 102 from contacting the skin of the babies directly, possibly causing an uncomfortable feeling.

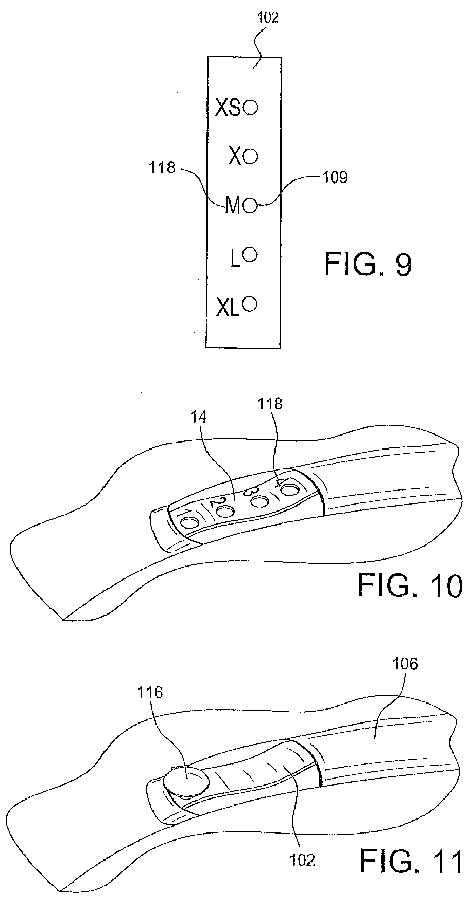

Each of the intermediate strips 101, 102 can also include a measuring indicator 118 (refer to Figs. 8-10), when the intermediate hole 109 is fastened onto the intermediate button (e.g., 104), the measuring indicator 118 is placed beside the intermediate button to show the currently used size of the legline. A user can adjust the position of the elastic strips 101, 102 to fit their baby perfectly by simply changing the elastic position. The measuring indicator 118 can include numbers, letters, scale marks or any other indication that can show the size setting of the intermediate strip.

The diaper sheath can also include a back elastic strip 125 attached to the inner and outer panels along the edge of the back portion that can make the intermediate portion 14 and back portion 12 adjustable.

The diaper sheath according to various embodiments may further comprise a back button attached along the edge of the back portion. The back elastic strip can have at least one button holes cooperatively fastenable with the back button.

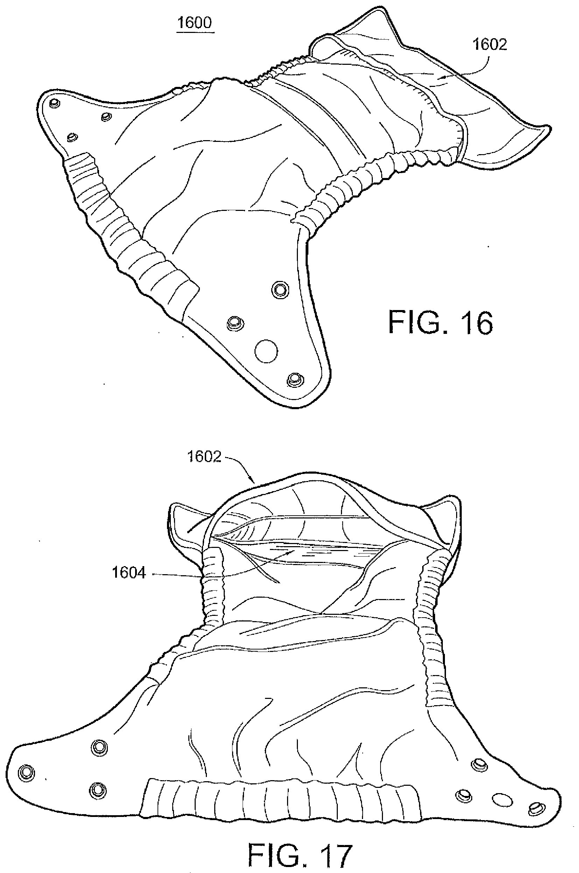

In addition, the diaper sheath can include a back flap attached to the back portion of the inner panel. Through the front flap and the back flap, the absorbent pad 15 can be held better. Similarly, in other embodiments, the back flap can also be used to cover the intermediate strip or the intermediate pockets opening, to prevent the directly contacting to the skin of the babies (see, e.g., Figs. 16-19). The diaper can also include a back opening covered by the back flap.

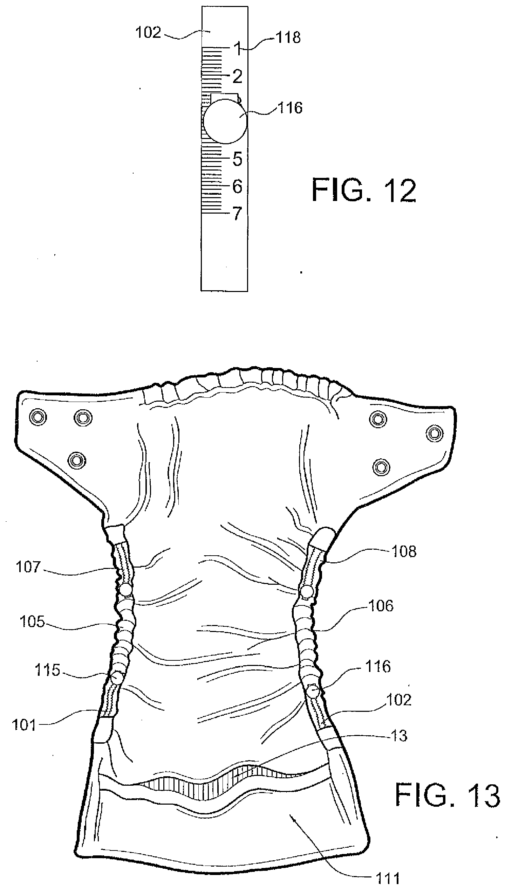

As shown in Fig. 13, there are two intermediate pocket openings 107, 108 on each of the intermediate pockets 105, 106. Both ends of the intermediate strips 101, 102 can be loosely hidden inside the intermediate pockets 105, 106, and can go through the intermediate pocket openings 107, 108. Two stoppers 115,116 are equipped on each of the intermediate strips 101, 102, which extend through the stoppers 115, 116. The stoppers 115, 116 can slide along the intermediate strips 101, 102 and stick onto the intermediate pocket opening 107, 108. During usage, the parents of the babies can adjust the position of the stoppers 115, 116 to tighten or release the intermediate strips 101,102, so the elasticity of the intermediate strips 101, 102 can be adjusted.

The stopper 115, 116 can be a spring-loaded stopper or toggle. When a button on the stopper is pressed down, the stopper can freely slide along the intermediate strip; and when the button is released, the stopper grips the intermediate strip under force of a spring-loaded mechanism and the position of the stopper (and the effective length of the intermediate strip) is fixed. However, in various embodiments, the intermediate strip is not detachable from the stopper, and vice versa.

The stoppers 115, 116 and the intermediate strips 101, 102 can be covered by intermediate pocket flaps 110, a front flap 111 or a back flap so that the stoppers 115, 116 will not contact the skin of the wearer directly. The intermediate pocket openings 107, 108 can be formed narrow so that the intermediate strips 101, 102 will not be exposed outside the intermediate pockets 105, 106, and the stoppers 115, 116 can be set inside the intermediate pockets 105, 106 as well.

In another embodiment of this invention, one end of each intermediate strip 101, 102 is fixed inside the intermediate pockets 105, 106, while the other end is loosely hidden inside the intermediate pockets 105, 106. In a further embodiment of this invention, both ends of each intermediate strip 101, 102 can be fixed within the intermediate pockets 105, 106.

In this embodiment, the measuring indicator is s series of scale marks on the intermediate strips 101, 102 (refer to Fig. 12). A user of the diaper can adjust the diaper quickly and accurately using the scale marks. Of course, the scale marks can be numbers, letters or other indicia as discussed above regarding Figs. 8-10.

Comparing with the intermediate button 103, 104 and the intermediate holes 109, the setting of the stoppers 115, 116 has the advantage that the leg opening adjustment can be more flexible, and is not restricted by the distance internals between the intermediate holes 109. In other words, the stoppers provide for continuously adjustable intermediate strips.

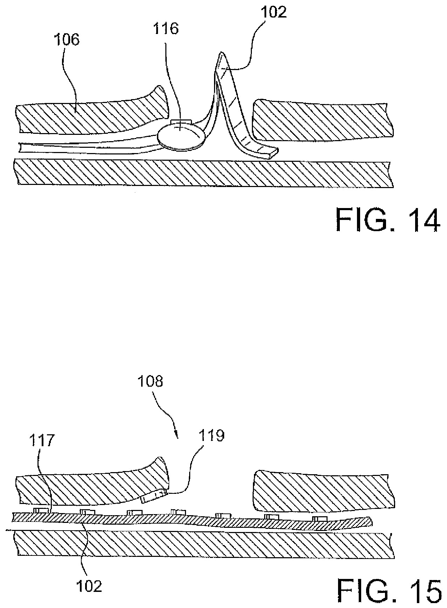

As shown in Fig. 15, in some embodiments the intermediate button 103, 104 is replaced by a front snap 119, and the intermediate holes 109 are replaced by plural back snaps 117 on each of intermediate strips 101, 102. The intermediate strips can adjust the leg openings of the diaper sheath by fastening the front snap 119 to a desired one of the back snaps 117.

Figs. 16-19 show an embodiment in which the intermediate strips are disposed under a front flap, which can prevent the intermediate strips from contacting the skin of the babies directly and possibly causing discomfort. Figs. 16 and 17 show a diaper 1600 with a front flap 1602 covering a front opening 1604.

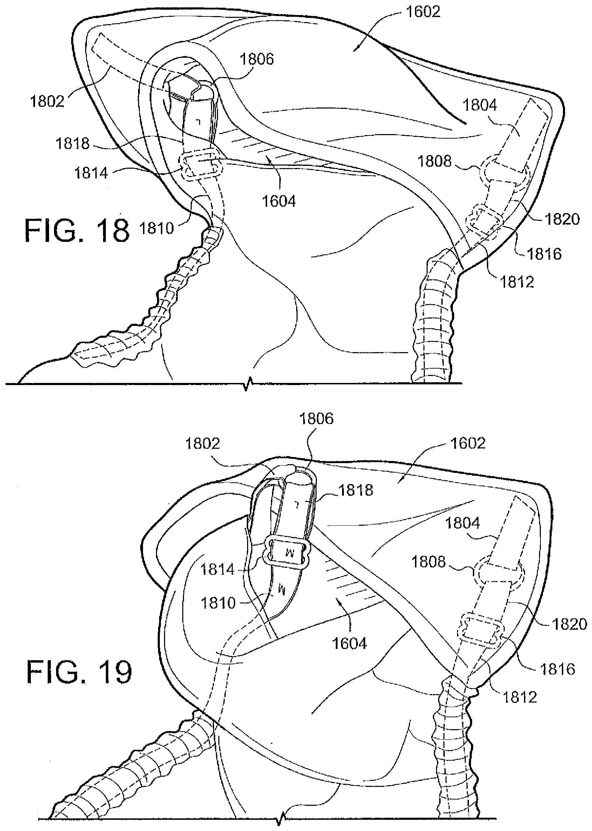

Fig. 18 shows anchor intermediate strip portions 1802 and 1804 attached to respective rings 1806 and 1808. Elongated intermediate strip portions 1810 and 1812 extend through respective clips 1814 and 1816 several times to form loops 1818 and 1820. Loops 1818 and 1820 can be changed in size/ length by moving elongated intermediate strip portions (1810, 1812) through respective clips (1814, 1816) in different directions, in a well known manner. However, the elongated intermediate strip portions 1810, 1812 are not detachable from the clips 1814, 1816 or the loops 1818, 1820. By using the looped strip and slide adjustment mechanism, a diaper in accordance with at least one embodiment can provide continuously adjustable diaper leg openings to provide a true "one size fits all" diaper. Also, as an alternative to the clips shown, other kinds of clips, e.g. a clip similar to the front part of the crocodile clip could be used. The clips can be relatively thin. Moreover, each leg opening is independently adjustable and can accommodate a wearer having different sized legs.

The intermediate strips used in some embodiments (e.g., the stopper, and loop and slide embodiments, or any not incorporating a button fastener) can be formed from elastic strips that have no holes or openings. The placement and shape of the rings 1806 and 1808 is an example for illustration purposes. It will be appreciated that the rings can be other shapes (such as D, or square) and also placed in other locations.

As shown in Fig. 19, an anchor intermediate strip and a corresponding elongated intermediate strip with clip can be temporarily pulled from the front opening 1604 to facilitate with adjustment and then returned to the front opening 1604 and covered by the front flap 1602.

It will be appreciated that while Figs. 18 and 19 show a slide and looped strip adjustment member, the adjustment member can be a spring-loaded stopper, spring-loaded toggle or other suitable adjustment device. Also, the stopper may be a non-spring-loaded stopper.

In at least one other embodiment, the elongated intermediate strip portions 1810 and 1812, clips 1814 and 1816, and loops 1818 and 1820 are disposed on sides of intermediate portion 14.

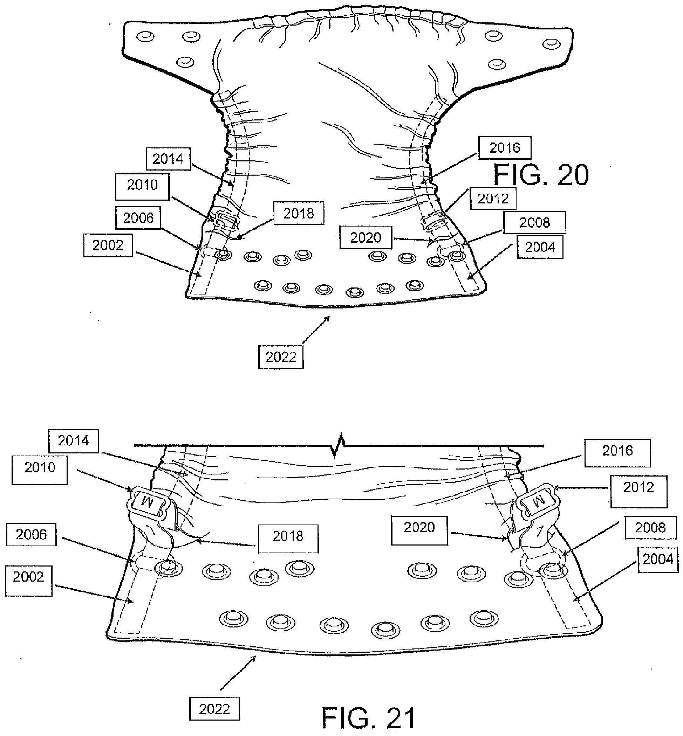

Figs. 20 and 21 are views of an exemplary diaper sheath showing continuously adjustable leg opening adjustment members disposed for access from exterior front openings in accordance with at least one embodiment. In particular, the diaper sheath shown in Figs. 20 and 21 includes shows anchor intermediate strip portions 2002 and 2004 attached to respective rings 2006 and 2008. Elongated intermediate strip portions 2014 and 2016 extend through respective clips 2010 and 2012 several times to form loops that wrap around each respective ring. The clips 2010 and 2012 can be accessed through slots 2018 and 2020, respectively. The loops can be changed in size/length by moving elongated intermediate strip portions (2014, 2016) through respective clips (2010, 2012) in different directions, in a well known manner. However, the elongated intermediate strip portions 2014, 2016 are not detachable from the clips 2010, 2012 or the loops. By using the looped strip and slide adjustment mechanism, a diaper in accordance with at least one embodiment can provide continuously adjustable diaper leg openings to provide a true "one size fits all" diaper. Moreover, each leg opening is independently adjustable and can accommodate a wearer having different sized legs.

In contrast to the embodiment shown in Figs. 18 and 19, the embodiment shown in Figs. 20 and 21 has the intermediate strips and adjustment clips disposed on the front, exterior side 2022 of the diaper sheath. In addition to being accessible through the slots, an embodiment with front accessible intermediate strips could include a flap and opening similar to that shown in Figs. 16-19 to cover the intermediate strips.

Fig. 21 shows how the intermediate strips and clips can be temporarily pulled through the slots 2018 and 2020 in order to adjust the lengths of the intermediate strips.

The intermediate strips used in some embodiments (e.g., the stopper, and loop and slide embodiments, or any not incorporating a button fastener) can be formed from elastic strips that have no holes or openings.

It will be appreciated that the openings, slots or access panels for the intermediate strip adjustment clips can be located on the interior or exterior of the diaper sheath and also disposed at any point along the intermediate strip or leg opening from the front to the back of the diaper sheath.

While the invention has been described in conjunction with a number of embodiments, it is evident that many alternatives, modifications and variations would be or are apparent to those of ordinary skill in the applicable arts. Accordingly, Applicant intends to embrace all such alternatives, modifications, equivalents and variations that are within the scope of the invention.

## Claims

1. A diaper sheath comprising:
a shell having an inner panel and an outer panel, each of said inner and outer panels having a front portion and a back portion, each of said panels attached peripherally one to the other in matched contour to form a front edge, a back edge, and a first side edge and a second side edge;
a first intermediate pocket and a second intermediate pocket set along the first side edge and the second side edge respectively, each intermediate pocket having at least one opening; and
a first intermediate strip and a second intermediate strip set inside each of the first and second intermediate pockets respectively, the first and second intermediate strips each having an anchor portion attached to an attachment ring, and an elongated portion having a loop formed through a slide, the loop passing through the attachment ring and having a continuously adjustable length selected by moving the slide to a desired position along the elongated portion.

2. The diaper sheath of claim 1, wherein the openings are set in a substantially middle portion of the intermediate pocket.

3. The diaper sheath of claim 1, wherein a portion of said intermediate strip is exposed outside said intermediate pockets through the intermediate pocket openings.

4. The diaper sheath of claim 3, further comprising intermediate pocket flaps positioned at the opening(s) of the intermediate pocket, said intermediate pocket openings are covered by said intermediate pocket flaps.

5. The diaper sheath of claim 1, wherein there are two intermediate pocket openings on each intermediate pocket.

6. The diaper sheath of claim 1, wherein said front portion of the inner panel has a front opening disposed thereon, and/or said back portion of the inner panel has a back opening.

7. The diaper sheath of claim 6, further comprising a front flap attached to said front portion of said inner panel, wherein said front opening is covered by said front flap.

8. The diaper sheath of claim 1, wherein said back portion of the inner panel has a back opening and further comprising a back flap attached to said back portion of said inner panel and covering said back opening.

9. The diaper sheath of claim 1, wherein the slide is a spring-loaded stopper and each intermediate strip is inserted through a respective stopper so that the position of the intermediate strip can be continuously adjusted.

10. The diaper sheath of claim 9, wherein the stoppers are placed within the intermediate pocket or disposed at the opening of the intermediate pocket.

11. The diaper sheath of claim 1, wherein said inner panel utilizes a laminated liquid permeable material and said outer panel utilizes a laminated liquid impervious material.

12. The diaper sheath of claim 1, further comprising a back elastic strip attached to said inner and outer panels along the edge of said back portion.

13. The diaper sheath of claim 12, further comprising an adjustment member slidably engaging said back elastic strip.

14. The diaper sheath of claim 1, further comprising two measuring indicators each disposed on a respective intermediate strip.

15. The diaper sheath of claim 1, further comprising a pad fully or partially inserted between said inner and outer panels, wherein said pad is a removable, reusable, absorbent pad, or wherein said pad is disposable.

## Patentansprüche

1. Windelhülle, umfassend:
eine Schale, die ein inneres Paneel und ein äußeres Paneel aufweist, wobei jedes von dem inneren und dem äußeren Paneel einen vorderen Abschnitt und einen hinteren Abschnitt aufweist, wobei jedes der Paneele an dem anderen in übereinstimmender Kontur umfangsmäßig befestigt ist, um eine Vorderkante, eine Hinterkante und eine erste Seitenkante und eine zweite Seitenkante zu bilden;
eine erste Zwischentasche und eine zweite Zwischentasche, gesetzt entlang der ersten Seitenkante bzw. der zweiten Seitenkante, wobei jede Zwischentasche zumindest eine Öffnung aufweist; und
einen ersten Zwischenstreifen und einen zweiten Zwischenstreifen, gesetzt im Inneren der ersten bzw. der zweiten Zwischentasche, wobei der erste und der zweite Zwischenstreifen jeweils einen Verankerungsabschnitt aufweist, der an einem Befestigungsring befestigt ist, und einen länglichen Abschnitt, der eine Schlaufe aufweist, die durch einen Schlitten gebildet ist, wobei die Schlaufe durch den Befestigungsring verläuft und eine kontinuierliche, anpassbare Länge aufweist, die durch Bewegen des Schlittens zu einer gewünschten Position entlang des länglichen Abschnitts ausgewählt wird.

2. Windelhülle nach Anspruch 1, wobei die Öffnungen in einem im Wesentlichen mittleren Abschnitt der Zwischentasche gesetzt sind.

3. Windelhülle nach Anspruch 1, wobei ein Abschnitt des Zwischenstreifens außerhalb der Zwischentasche durch die Zwischentaschenöffnungen freiliegend ist.

4. Windelhülle nach Anspruch 3, weiter umfassend Zwischentaschenklappen, angeordnet an der Öffnung (den Öffnungen), wobei die Zwischentaschenöffnungen von den Zwischentaschenklappen bedeckt sind.

5. Windelhülle nach Anspruch 1, wobei es zwei Zwischentaschenöffnungen an jeder Zwischentasche gibt.

6. Windelhülle nach Anspruch 1, wobei der vordere Abschnitt des inneren Paneels eine darauf angeordnete vordere Öffnung aufweist und/oder der hintere Abschnitt des inneren Paneels eine darauf angeordnete hintere Öffnung aufweist.

7. Windelhülle nach Anspruch 6, weiter umfassend eine Vorderklappe, die an dem vorderen Abschnitt des inneren Paneels befestigt ist, wobei die vordere Öffnung von der Vorderklappe bedeckt ist.

8. Windelhülle nach Anspruch 1, wobei der hintere Abschnitt des inneren Paneels eine hintere Öffnung aufweist und weiter umfassend eine Hinterklappe, die an dem hinteren Abschnitt des inneren Paneels befestigt ist und die hintere Öffnung bedeckt.

9. Windelhülle nach Anspruch 1, wobei der Schlitten ein federbelasteter Anschlag ist und jeder Zwischenstreifen durch einen entsprechenden Anschlag eingesetzt wird, sodass die Position des Zwischenstreifens kontinuierlich angepasst werden kann.

10. Windelhülle nach Anspruch 9, wobei die Anschläge innerhalb der Zwischentasche platziert sind oder an der Öffnung der Zwischentasche angeordnet sind.

11. Windelhülle nach Anspruch 1, wobei das innere Paneel ein laminiertes, flüssigkeitsdurchlässiges Material verwendet und das äußere Paneel ein laminiertes flüssigkeitsundurchlässiges Material verwendet.

12. Windelhülle nach Anspruch 1, weiter umfassend einen hinteren elastischen Streifen, der an dem inneren und dem äußeren Paneel entlang der Kante des hinteren Abschnitts befestigt ist.

13. Windelhülle nach Anspruch 12, weiter umfassend ein Anpassungselement, das verschiebbar mit dem hinteren elastischen Streifen im Eingriff ist.

14. Windelhülle nach Anspruch 1, weiter umfassend zwei Messindikatoren, von denen jeder auf einem entsprechenden Zwischenstreifen angeordnet ist.

15. Windelhülle nach Anspruch 1, weiter umfassend ein Pad, das vollständig oder teilweise zwischen dem inneren Paneel und dem äußeren Paneel eingesetzt ist, wobei das Pad ein entfernbares, wiederverwendbares, absorbierendes Pad ist oder wobei das Pad wegwerfbar ist.

## Revendications

1. Gaine de couche comprenant :
une enveloppe ayant un panneau intérieur et un panneau extérieur, chacun desdits panneaux intérieur et extérieur ayant une portion avant et une portion arrière, chacun desdits panneaux étant fixé de façon périphérique l'un à l'autre en contour apparié pour former un bord avant, un bord arrière, et un premier bord latéral et un second bord latéral ;
une première poche intermédiaire et une seconde poche intermédiaire situées le long du premier bord latéral et du second bord latéral respectivement, chaque poche intermédiaire ayant au moins une ouverture ; et
une première bande intermédiaire et une seconde bande intermédiaire situées à l'intérieur de chacune des première et seconde poches intermédiaires respectivement, les première et seconde bandes intermédiaires ayant chacune une portion d'ancrage fixée à un anneau de fixation, et une portion allongée ayant une boucle formée à travers une glissière, la boucle passant à travers l'anneau de fixation et ayant une longueur réglable en continu choisie en déplaçant la glissière jusqu'à une position souhaitée le long de la portion allongée.

2. Gaine de couche selon la revendication 1, dans laquelle les ouvertures sont situées dans une portion sensiblement au milieu de la poche intermédiaire.

3. Gaine de couche selon la revendication 1, dans laquelle une portion de ladite bande intermédiaire est exposée à l'extérieur desdites poches intermédiaires à travers les ouvertures de poche intermédiaire.

4. Gaine de couche selon la revendication 3, comprenant en outre des rabats de poche intermédiaire positionnés au niveau de l'/des ouverture(s) de la poche intermédiaire, lesdites ouvertures de poche intermédiaire sont recouvertes par lesdits rabats de poche intermédiaire.

5. Gaine de couche selon la revendication 1, dans laquelle se trouvent deux ouvertures de poche intermédiaire sur chaque poche intermédiaire.

6. Gaine de couche selon la revendication 1, dans laquelle ladite portion avant du panneau intérieur a une ouverture avant disposée sur celle-ci, et/ou ladite portion arrière du panneau intérieur a une ouverture arrière.

7. Gaine de couche selon la revendication 6, comprenant en outre un rabat avant fixé à ladite portion avant dudit panneau intérieur, dans laquelle ladite ouverture avant est recouverte par ledit rabat avant.

8. Gaine de couche selon la revendication 1, dans laquelle ladite portion arrière du panneau intérieur a une ouverture arrière et comprenant en outre un volet arrière fixé à ladite portion arrière dudit panneau intérieur et recouvrant ladite ouverture arrière.

9. Gaine de couche selon la revendication 1, dans laquelle la glissière est une butée à ressort et chaque bande intermédiaire est insérée à travers une butée respective de sorte que la position de la bande intermédiaire puisse être réglée en continu.

10. Gaine de couche selon la revendication 9, dans laquelle les butées sont placées au sein de la poche intermédiaire ou disposées au niveau de l'ouverture de la poche intermédiaire.

11. Gaine de couche selon la revendication 1, dans laquelle ledit panneau intérieur utilise un matériau stratifié perméable aux liquides et ledit panneau extérieur utilise un matériau stratifié imperméable aux liquides.

12. Gaine de couche selon la revendication 1, comprenant en outre une bande élastique arrière fixée auxdits panneaux intérieur et extérieur le long du bord de ladite portion arrière.

13. Gaine de couche selon la revendication 12, comprenant en outre un organe de réglage venant en prise en coulissement avec ladite bande élastique arrière.

14. Gaine de couche selon la revendication 1, comprenant en outre deux indicateurs de mesure disposés chacun sur une bande intermédiaire respective.

15. Gaine de couche selon la revendication 1, comprenant en outre un coussinet inséré totalement ou partiellement entre lesdits panneaux intérieur et extérieur, dans laquelle ledit coussinet est un coussinet amovible, réutilisable, absorbant, ou dans laquelle ledit coussinet est jetable.
